# EUROPEAN PATENT APPLICATION

(11) **EP 3 144 380 A1**
(43) Date of publication of application: **22.03.2017**
(21) Application number: 16743489.3
(22) Date of filing: 28.01.2016
(51) Int. Cl.: C12M 1/34, C12M 1/00, C12Q 1/68, G01N 35/08, G01N 37/00

(54) **ANALYSIS DEVICE, ANALYSIS CHIP, ANALYSIS KIT, AND ANALYSIS METHOD USING SAME**

(30) Priority: 28.01.2015 JP 2015014849
(71) Applicant: Kabushiki Kaisha Dnaform, Kanagawa 230-0046 (JP)
(72) Inventor: TSUJIMARU Koichiro, Yokohama-shi Kanagawa 230-0046 (JP)
(74) Representative: Tuxworth, Pamela M.
(86) International application number: PCT/JP2016/052530
(87) International publication number: WO 2016/121886

(57) **Abstract**

The present invention provides a tool that can analyze a target in a sample by a simple operation and can be downsized and an analysis method using the tool. By inserting an analysis chip into an analysis device, a reaction of a sample and a reagent is analyzed. The analysis chip includes a parallel flow channel in which plural reaction flow channels are connected in parallel, and the axial direction of the parallel flow channel is a direction of inserting the analysis chip into the analysis device. The analysis device includes a main body case being a housing, including an insertion opening into which the analysis chip is to be inserted; and a void; a heating unit that causes a sample to thermally react with a reagent, the heating unit being disposed in the void at at least one of inner surfaces so as to face a parallel surface of the parallel flow channel of the analysis chip; a light source unit that emits light to the analysis chip, the light source unit being disposed in the void at least above or below a reaction flow channel located at at least one end of the parallel flow channel of the analysis chip and extended along the axial direction of the parallel flow channel; and a plurality of detection units that detect the thermal reactions, each detection unit being disposed in the void at a downstream side end in the insertion direction of the analysis chip so as to correspond to each reaction flow channel of the analysis chip.

## Description

### Technical Field

The present invention relates to an analysis device, an analysis chip, an analysis kit, and an analysis method using the same.

### Background Art

Nowadays, detection of the target gene of the source of infection in a biological sample is a common method for inspecting infectious diseases due to viruses, bacteria, and the like. The detection of the target gene is commonly carried out by pretreating a collected biological sample, amplifying the nucleic acid of the target gene in the pretreated biological sample using a primer, and detecting the presence or absence or the amount of the nucleic acid amplification. The detection requires plural steps, a special apparatus, and the like. Thus, the detection of the target gene is carried out in a medical institution such as a hospital or the like or in a special inspection agency.

On the other hand, the fact is that there is a demand for the detection not in an inspection agency or the like but at a personal level as described below. For example, when a patient has cold symptoms, it is preferable if the patient can preliminarily test whether he/she has influenza also from the viewpoint of preventing secondary infection. Furthermore, among the infectious diseases, in particular, with reference to sexually transmitted diseases such as HIV, Candida, and the like, a patient having such a disease often hesitates to take a test in a hospital and therefore tends to discover the disease too late. Thus, if a patient can take a test at home, such a disease can be discovered early.

For making the test at home implementable, an operation should be easy and a device for use in the test should be small, and attempts are being made to develop such a device. However, a small device with an easy operation that can be used at a personal level has not been provided so far because the test requires all of pretreatment of a biological sample, mixing of the biological sample and a reagent for nucleic acid amplification, amplification reaction of the nucleic acid, and detection of the reaction.

### Brief Summary of the Invention

### Problem to be Solved by the Invention

Hence, the present invention is intended to provide, for example, a tool that can analyze a target in a sample by a simple operation and can be downsized, and an analysis method using the tool.

### Means for Solving Problem

In order to achieve the above object, the present invention provides an analysis device into which an analysis chip is to be inserted for analyzing a reaction of a sample and a reagent in the analysis chip, the analysis chip to be inserted into the analysis device including a parallel flow channel in which plural reaction flow channels are connected in parallel, an axial direction of the parallel flow channel being a direction of inserting the analysis chip into the analysis device. The analysis device includes a main body case being a housing, including an insertion opening into which the analysis chip is to be inserted; and a void in communication with the insertion opening; a heating unit that causes a sample to thermally react with a reagent in the analysis chip, the heating unit being disposed in the void at at least one of inner surfaces so as to face a parallel surface of the parallel flow channel of the analysis chip in a state where the analysis chip is inserted in the analysis device; a light source unit that emits light to the analysis chip, the light source unit being disposed in the void at least above or below a reaction flow channel located at at least one end of the parallel flow channel of the analysis chip and extended along the axial direction of the parallel flow channel in the state where the analysis chip is inserted in the analysis device; and a plurality of detection units that detect the thermal reactions of the samples and the reagents in the analysis chip, each detection unit being disposed in the void at a downstream side end in the insertion direction of the analysis chip so as to correspond to each reaction flow channel of the analysis chip in the state where the analysis chip is inserted in the analysis device.

The present invention also provides an analysis chip to be inserted into an analysis device, including: a parallel flow channel in which plural reaction flow channels are connected in parallel.

The present invention also provides an analysis chip to be inserted into an analysis device, including: a parallel flow channel in which plural reaction flow channels are connected in parallel; a pair of parallel pistons; and a reagent. Each flow channel of the parallel flow channel includes a reagent therein. Each flow channel includes: a first syringe region; and a second syringe region. A tip of the first syringe region is connected to a tip of the second syringe region, and the flow channel can be cut in a vicinity of a connection site of the first syringe region and the second syringe region. The pair of parallel pistons includes: a first parallel piston in which plural pistons are connected in parallel; and a second parallel piston in which plural pistons are connected in parallel. Each piston of the first parallel piston can be inserted into each flow channel from an end side of the first syringe region of the parallel flow channel, each piston of the second parallel piston can be inserted into each flow channel from an end side of the second syringe region of the parallel flow channel, and after the sample and the reagent have mixed to prepare a reaction system, the parallel flow channel at a first syringe region side is used as a reaction detection unit.

The present invention also provides an analysis kit including the analysis device according to the present invention and the analysis chip according to the present invention.

The present invention also provides an analysis method of a sample including the following steps: introducing a sample into the analysis chip according to the present invention; mixing the sample and the reagent in the analysis chip; inserting the analysis chip into the analysis device according to the present invention; causing the sample to thermally react with the reagent in the analysis chip by the heating unit of the analysis device; and detecting the reaction in the analysis chip by the detection unit of the analysis device.

### Effects of the Invention

According to the present invention, for example, an analysis device can be downsized. Furthermore, the present invention allows, for example, an analyst to analyze a target in a sample easily by preliminarily mixing a sample and a reagent using the analysis chip of the present invention to prepare a reaction system and then simply inserting the analysis chip into the analysis device of the present invention. Thus, for example, viral infection due to an influenza virus and the like and sexually transmitted diseases such as HIV, chlamydia, and the like can be analyzed easily. In particular, the present invention allows an analyst to analyze a target easily by himself/herself without going to an inspection agency such as a hospital or the like, for example, because the analysis device can be downsized.

### Brief Description of Drawings

[FIG. 1] FIG. 1A is a perspective view schematically showing an example of an analysis device of the present invention; FIG. 1B is a cross sectional view taken along the line I-I in FIG. 1A; and FIG. 1C is a cross sectional view taken along the line II-II in FIG. 1A.
[FIG. 2] FIG. 2 is a schematic view showing the direction of the light emitted from light source units in the analysis device of the present invention.
[FIG. 3] FIG. 3 is a cross sectional view schematically showing an example of an analysis chip of the present invention.
[FIG. 4] FIGs. 4A and 4B are cross sectional views of a part of the analysis chip of the present invention schematically showing the usage of the analysis chip; and FIG. 4C is a cross sectional view showing the state where the analysis chip is separated.
[FIG. 5] FIG. 5 is a perspective view schematically showing an example of the analysis chip of the present invention.
[FIG. 6] FIG. 6 is a perspective view schematically showing an example of the analysis chip of the present invention.
[FIG. 7] FIG. 7 shows perspective views schematically showing an example of the usage of the analysis device and analysis chip of the present invention.
[FIG. 8] FIG. 8 shows schematic views showing the direction of the light in the state where the analysis chip of the present invention is set in the analysis device of the present invention.
[FIG. 9] FIG. 9 is a plan view schematically showing an example of an analysis chip of the present invention.
[FIG. 10] FIGs. 10A and 10B are views schematically showing an example of the usage of the analysis chip of the present invention.
[FIG. 11] FIG. 11 shows schematic views showing an example of an analysis method using the analysis chip and analysis device of the present invention.

### Mode for Carrying out the Invention

The present invention is described in more detail below with reference to examples. The present invention, however, is not limited by the following description.

The present invention relates to an analysis device, an analysis chip, an analysis kit, and an analysis method using them. According to the present invention, for example, the analysis method of the present invention can be performed by mixing a sample and a reagent to prepare a reaction system using the analysis chip of the present invention and then simply setting the analysis chip in the analysis device of the present invention.

### [Analysis device]

As described above, the analysis device of the present invention is an analysis device into which an analysis chip is to be inserted for analyzing a reaction of a sample and a reagent in the analysis chip, the analysis chip to be inserted into the analysis device including a parallel flow channel in which plural reaction flow channels are connected in parallel, an axial direction of the parallel flow channel being a direction of inserting the analysis chip into the analysis device. The analysis device includes a main body case being a housing, including an insertion opening into which the analysis chip is to be inserted; and a void in communication with the insertion opening; a heating unit that causes a sample to thermally react with a reagent in the analysis chip, the heating unit being disposed in the void at at least one of inner surfaces so as to face a parallel surface of the parallel flow channel of the analysis chip in a state where the analysis chip is inserted in the analysis device; a light source unit that emits light to the analysis chip, the light source unit being disposed in the void at least above or below a reaction flow channel located at at least one end of the parallel flow channel of the analysis chip and extended along the axial direction of the parallel flow channel in the state where the analysis chip is inserted in the analysis device; and a plurality of detection units that detect the thermal reactions of the samples and the reagents in the analysis chip, each detection unit being disposed in the void at a downstream side end in the insertion direction of the analysis chip so as to correspond to each reaction flow channel of the analysis chip in the state where the analysis chip is inserted in the analysis device.

While the analysis chip that is to be inserted into the analysis device is described in the description with reference to the analysis device of the present invention, this is merely for describing the positional relationship between the analysis device and the analysis chip and this is not intended to describe that the analysis device of the present invention is provided with the analysis chip as an essential component. Thus, hereinafter, the "analysis chip" in the description with reference to the analysis device of the present invention denotes the position of the analysis chip in the state where the analysis chip is inserted in the analysis device of the present invention, unless otherwise noted.

In the analysis device of the present invention, the form of the main body case is a housing. The material for the main body case is not limited to a particular material and the material can be, for example, a plastic member.

In the analysis device of the present invention, the heating unit is not limited to a particular type, and the heating unit can be any heating unit as long as it can heat the analysis chip to be inserted. For example, a heater can be used as the heating unit.

Preferably, the analysis device of the present invention further includes a heat insulation section, for example. For example, the heating unit is disposed in the void of the main body case through the heat insulation section. In the analysis device of the present invention, the heat insulation section may be disposed only in a region where the heating unit is disposed. For example, the heat insulation section may be disposed also outside the region where the heating unit is disposed because it allows sufficient heat insulation performance. As a specific example, the heat insulation section may be disposed in the whole inner region of the main body case. The heat insulation section is not limited to a particular type, and a common heat insulation material can be used.

In the analysis device of the present invention, preferably, the light source unit includes a linear light source; and a converter that converts linear light into surface light, wherein the linear light source unit emits linear light along an axial direction of the reaction flow channel, and the converter convers the linear light into surface light and emits the surface light, for example. Because the linear light emitted along the axial direction of the reaction flow channel is converted into surface light, when the analysis chip is inserted in the analysis device of the present invention, the parallel surface of the parallel flow channel of the analysis chip can be irradiated with the surface light. In other words, in the analysis device of the present invention, although the linear light source is disposed in the vicinity of the reaction flow channel at an end of the analysis chip, the entire parallel flow channel of the analysis chip can be irradiated with light more effectively.

For example, in the void of the main body case, the light source unit may be disposed only above the reaction flow channel at one end or the other end of the parallel flow channel of the analysis chip, the light source unit may be disposed only below the reaction flow channel at one end or the other end of the parallel flow channel of the analysis chip, the light source units may be disposed only above or below the reaction flow channels at both ends of the parallel flow channel of the analysis chip, or the light source units may be disposed above the reaction flow channel at one end of the parallel flow channel and below the reaction flow channel at the other end of the parallel flow channel. When the light source units are disposed at the reaction flow channels at both ends of the parallel flow channel, preferably, the light source units are disposed such that the direction of the linear light emitted from one light source unit is in the opposite direction to the direction of the linear light emitted from the other light source unit.

There is no particular limitation on the type of the light emitted from the light source unit. Also, the light source unit is not limited to a particular type, and an LED, an optical fiber, and the like can be used, for example. The type of the light source unit can be determined appropriately according to the type of the reagent for the target, for example. Preferably, the light source unit can emit excitation light suitable for the reagent.

The analysis device of the present invention includes a plurality of detection units for separately detecting the reactions of the samples and the reagents in the reaction flow channels of the analysis chip. Each detection unit is disposed so as to correspond to each reaction flow channel as described above.

In the analysis device of the present invention, the detection unit is not limited to a particular unit, and the detection unit can be, for example, a charge coupled device (CCD) and the like. Preferably, the detection unit can detect luminescence, fluorescence, and the like that are generated by the direct or indirect reaction of a reagent and a target in a sample, for example.

The analysis device of the present invention further includes: a power supply; or a connector to be connected to an external power supply, for example. The external power supply is not limited to a particular power supply, and examples thereof include a personal computer, a tablet, and a cellular phone. The connector to be connected to an external power supply can be, for example, a universal serial bus (USB) connector and the like.

The application of the analysis device of the present invention is not limited to a particular application. For example, the analysis device of the present invention can be used for nucleic acid analysis to be described below.

### [First analysis chip]

As described above, the analysis chip of the present invention is an analysis chip to be inserted into an analysis device. The analysis chip includes a parallel flow channel in which plural reaction flow channels are connected in parallel.

Preferably, the parallel flow channel of the analysis chip of the present invention has a translucent parallel surface, and a side surface of each reaction flow channel that is in contact with an adjacent reaction flow channel in the parallel flow channel has light blocking ability, for example. When a sample is analyzed by inserting the analysis chip of the present invention into the analysis device of the present invention, as described above, the reaction in each reaction flow channel of the analysis chip is detected by each of the detection units of the analysis device. Thus, for example, there is a possibility that the detection unit detects not only the reaction of a corresponding reaction flow channel but also the reaction of an adjacent reaction flow channel. However, according to the above described configuration of the parallel flow channel, because the side surface between the flow channels has light blocking ability, the reaction of an adjacent reaction flow channel can be prevented from being detected by the detection units, whereby the detection accuracy can further be increased.

Preferably, in the analysis chip of the present invention, the parallel flow channel is configured to close the openings at both ends of the reaction flow channels, for example. The method of closing the parallel flow channel is not limited to a particular method, and, for example, the parallel flow channel may be closed by thermal adhesion or each opening of the parallel flow channel may be closed with a cap.

In the analysis chip of the present invention, for example, each reaction flow channel of the parallel flow channel may include a condenser lens therein and may further include an optical fiber therein. Such an analysis chip can detect the reaction in accordance with the distance in the reaction flow channel of the analysis chip, for example. Thus, the position where the reaction is caused in the reaction flow channel can be analyzed. This allows scanning in the reaction flow channel and also allows counting of the target in the sample, for example.

In the analysis chip of the present invention, preferably, each reaction flow channel of the parallel flow channel includes a reagent therein, for example. The reagent is not limited to a particular reagent, and the reagent includes an analysis reagent that reacts with a target in a sample. In this case, preferably, at least two reaction flow channels of the parallel flow channel include analysis reagents that react with different targets in the sample because it allows an analyst to analyze different targets in the reaction flow channels of the parallel flow channel, for example.

The target to be analyzed using the analysis chip of the present invention is not limited to a particular target, and the target can be, for example, a nucleic acid as described below. When the target is a nucleic acid, in the analysis chip of the present invention, preferably, the analysis reagent includes a reagent for nucleic acid amplification, for example. Also, when the target is a nucleic acid, in the analysis chip of the present invention, preferably, the reagent includes a reagent for nucleic acid detection, for example. The reagent for nucleic acid detection is not limited to a particular reagent, and the reagent can be, for example, a fluorescent reagent. Also, the reagent may include a pretreatment reagent of a sample, for example. The pretreatment reagent is not limited to a particular type, and the pretreatment reagent can be determined appropriately according to the type of the sample, for example.

### [Second analysis chip]

As described above, the analysis chip of the present invention is an analysis chip to be inserted into an analysis device, including: a parallel flow channel in which plural reaction flow channels are connected in parallel; a pair of parallel pistons; and a reagent. Each flow channel of the parallel flow channel includes a reagent therein. Each flow channel includes: a first syringe region; and a second syringe region. The tip of the first syringe region is connected to the tip of the second syringe region, and the flow channel can be cut in a vicinity of a connection site of the first syringe region and the second syringe region. The pair of parallel pistons includes: a first parallel piston in which plural pistons are connected in parallel; and a second parallel piston in which plural pistons are connected in parallel. Each piston of the first parallel piston can be inserted into each flow channel from an end side of the first syringe region of the parallel flow channel, each piston of the second parallel piston can be inserted into each flow channel from an end side of the second syringe region of the parallel flow channel, and after the sample and the reagent have mixed to prepare a reaction system, the parallel flow channel at a first syringe region side is used as a reaction detection unit.

In the analysis chip of the present invention, as described above, the flow channel has a configuration in which the tip of the first syringe region is connected to the tip of the second syringe region. For example, two separate syringes may be connected such that the interiors thereof are in communication with each other or the first syringe region and the second syringe region may be molded in one piece.

In the parallel flow channel in which plural reaction flow channels are connected in parallel in the analysis chip of the present invention, for example, one of the parallel flow channel at the first syringe region side and the parallel flow channel at a second syringe region side is to be used as a flow channel for reagent arrangement and the other of the parallel flow channel at the first syringe region side and the parallel flow channel at the second syringe region side is to be used as a flow channel for sample introduction. In the analysis chip of the present invention, the parallel flow channel at either region side may be the flow channel for reagent arrangement or the flow channel for sample introduction.

In the analysis chip of the present invention, the parallel flow channel at the first syringe region side to be used as the reaction detection unit has a translucent parallel surface, and a side surface of each flow channel that is in contact with an adjacent flow channel in the parallel flow channel has light blocking ability, for example. For the same reason described with reference to the first analysis chip of the present invention, because the side surface between the flow channels has light blocking ability, such a configuration prevents the reaction of an adjacent reaction flow channel from being detected by the detection units in the analysis using the analysis device of the present invention and allows further improvement in the detection accuracy.

In the analysis chip of the present invention, for example, after the sample and the reagent have mixed to prepare a reaction system, by cutting the parallel flow channel in a parallel direction, the parallel flow channel at the first syringe region side may be used as a reaction detection unit.

In the analysis chip of the present invention, the parallel flow channel at the first syringe region side to be used as the reaction detection unit may include a condenser lens therein. Also, the parallel flow channel at the first syringe region side to be used as the reaction detection unit may further include an optical fiber therein. The optical fiber may be contained in each piston of the first parallel piston to be inserted into the parallel flow channel at the first syringe region side to be used as the reaction detection unit, for example. For the same reason as described with reference to the first analysis chip of the present invention, such a configuration allows scanning in the reaction flow channel and also allows counting of the target in the sample, for example.

In the analysis chip of the present invention, the reagent is not limited to a particular reagent. In the analysis chip of the present invention, the reagent may be disposed in a dry form or in a liquid form, for example. In the former case, for example, by introducing a liquid sample into the analysis chip of the present invention as the sample, the reagent in a dry form and the sample may be mixed, or, by adding a solvent to the analysis chip of the present invention, the sample and the reagent may be mixed. The solvent is not limited to a particular solvent, and examples of the solvent include water, a buffer solution, physiological saline, and mixtures thereof.

The reagent may include a pretreatment reagent of a sample, for example. The pretreatment reagent can be determined appropriately according to the type of the sample, for example.

The reagent includes an analysis reagent that reacts with a target in a sample, for example. Preferably, the reagent is the one that generates luminescence, fluorescence, and the like by the direct or indirect reaction with the target, for example.

In the analysis chip of the present invention, preferably, at least two flow channels of the parallel flow channel include analysis reagents that react with different targets in the sample because it allows an analyst to analyze different targets in the reaction flow channels of the parallel flow channel, for example.

The target to be analyzed using the analysis chip of the present invention is not limited to a particular target, and the target can be, for example, a nucleic acid as described below. When the target is a nucleic acid, in the analysis chip of the present invention, preferably, the analysis reagent includes a reagent for nucleic acid amplification, for example. Examples of the reagent for nucleic acid amplification include a primer, polymerase, and components that can be used for nucleic acid amplification. The primer can be determined appropriately according to the type of the target, for example. The primer can be, for example, a labeled primer. As the labeled primer, for example, a primer that has an exciton effect described in Japanese Patent No. 4370385 and the like can be used.

When the target is a nucleic acid, preferably, the analysis chip of the present invention includes a reagent for nucleic acid detection, for example. The reagent for nucleic acid detection can be a probe and the like to a target. The probe may be labeled with a labeling substance, for example. The labeling substance is not limited to a particular substance, and the labeling substance can be, for example, a fluorescent material. As the labeled probe, for example, a probe that has an exciton effect described in Japanese Patent No. 4761086 and the like can be used. Also, as the reagent for nucleic acid detection, for example, an intercalator such as SYBR (registered trademark) Green or the like and a ruthenium complex can be used.

### [Analysis kit]

As described above, the analysis kit of the present invention includes the analysis device according to the present invention and the analysis chip according to the present invention.

According to the present invention, by preparing a reaction system using the analysis chip and inserting the analysis chip into the analysis device of the present invention, a target in a sample can be analyzed easily.

### [Analysis method]

As described above, the analysis method of the present invention includes the following steps: introducing a sample into the analysis chip according to the present invention; mixing the sample and the reagent in the analysis chip; inserting the analysis chip into the analysis device according to the present invention; causing the sample to thermally react with the reagent in the analysis chip by the heating unit of the analysis device; and detecting the reaction in the analysis chip by the detection unit of the analysis device.

According to the present invention, by preparing a reaction system using the analysis chip and inserting the analysis chip into the analysis device of the present invention, a target in a sample can be analyzed easily.

In the introduction step of the analysis method of the present invention, preferably, the first parallel piston is inserted from an end of the parallel flow channel at the first syringe region side, a sample is introduced from an end of the parallel flow channel at the second syringe region side, and after the sample has introduced, the second parallel piston is inserted from the end of the parallel flow channel at the second syringe region side, or the second parallel piston is inserted from an end of the parallel flow channel at the second syringe region side, a sample is introduced from an end of the parallel flow channel at the first syringe region side, and after the sample has been introduced, the first parallel piston is inserted from the end of the parallel flow channel at the first syringe region side, for example.

In the analysis method of the present invention, the method of mixing the sample and the reagent in the mixing step is not limited to a particular method, and the method can be a method using a piston and a syringe. That is, in the mixing step of the analysis method of the present invention, for example, by alternately pushing the first parallel piston and the second parallel piston, the sample and the reagent can be mixed in each flow channel of the parallel flow channel.

In the analysis method of the present invention, when the analysis chip is the second analysis chip, for example, after the mixing step and before the insertion step, by cutting the parallel flow channel of the analysis chip in a parallel direction, the parallel flow channel at the first syringe region side may be separated as a reaction detection unit, and the reaction detection unit may be inserted into the analysis device as the analysis chip. In the present invention, the method of cutting the parallel flow channel is not limited to a particular method, and the method can be determined appropriately according to the structure and the like of the parallel flow channel, for example.

In the analysis method of the present invention, the analysis target is not limited to a particular target. The target can be, for example, a nucleic acid. The nucleic acid is not limited to a particular type, and examples thereof include DNA, cDNA, and RNA. The RNA can be, for example, mRNA and miRNA. The origin of the nucleic acid is not particularly limited, and examples thereof include viruses, bacteria, and mold. Examples of the virus include various influenza viruses, HIV, and herpes. Examples of the bacterium include chlamydia, Neisseria gonorrhoeae, and Treponema (syphilis). Examples of the mold include fungi such as Candida and the like.

In the analysis method of the present invention, the sample is not limited to a particular sample, and examples of the sample include animal-derived samples; plant-derived samples; environmental samples such as seawater, soil, drainage, and the like; and food and beverage samples such as drinking water, food, and the like. Examples of the animal-derived sample include biological samples such as blood (including whole blood and isolated blood cell); blood serum; blood plasma; cells (including cultured cells); tissues; body fluid (e.g., ear discharge, nasal discharge, pus, ascites, pleural fluid, bile, spinal fluid, expectoration, and the like); mucosa cells (e.g., oral mucosa cells, gastric mucosa cells, respiratory mucosa, and the like); swabs collected from nasal mucosa, oral mucosa, and the like using a cotton swab or the like; sweat; amniotic fluid; excreta (e.g., urine, feces, and the like); brushing collected from organs using an endoscope or the like; collected liquid; biopsy samples; alveolar washing liquid; and the like. In the analysis method of the present invention, preferably, the sample to be introduced into the analysis chip is a pretreated sample, for example.

Next, the present invention is described in detail with reference to drawings.

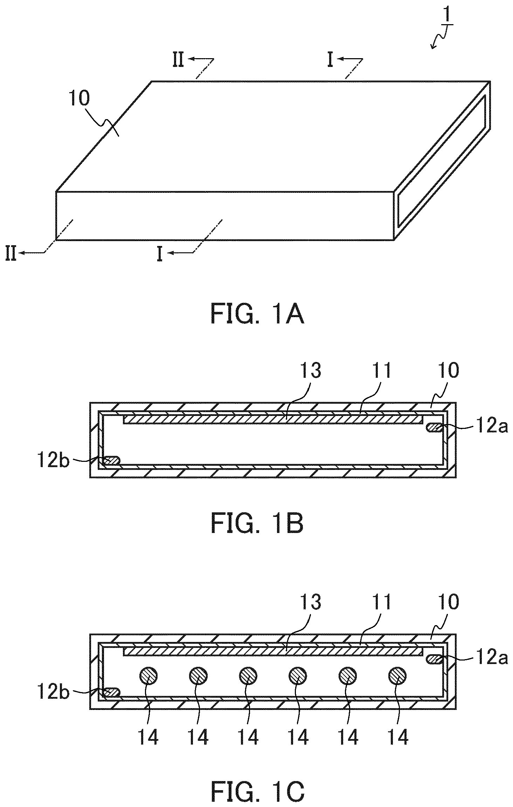

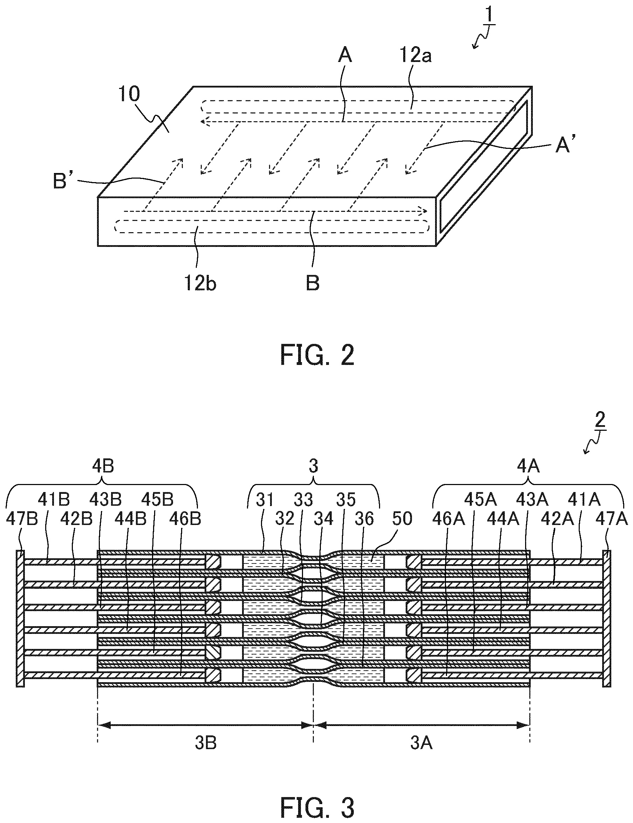

An example of the analysis device of the present invention is described with reference to FIGs. 1 and 2.

FIG. 1A is a perspective view schematically showing an example of an analysis device 1 of the present invention; FIG. 1B is a cross sectional view of the analysis device 1 taken along the line I-I in FIG. 1A; and FIG. 1C is a cross sectional view of the analysis device 1 taken along the line II-II in FIG. 1A.

The analysis device 1 includes a main body case 10, a heating unit 13, a heat insulation section 11, light source units 12a and 12b, and plural detection units 14. The main body case 10 is a housing. The main body case 10 includes an insertion opening into which the analysis chip that is described below is to be inserted and a void that is in communication with the insertion opening. Inside the main body case 10, the heat insulation section 11 is disposed, and the heating unit 13 is disposed through the heat insulation section 11. Specifically, the heating unit 13 is disposed so as to face the parallel surface of the parallel flow channel of the analysis chip in the state where the analysis chip is inserted in the analysis device 1. In the void of the main body case 10, the light source unit 12a is disposed above the reaction flow channel located at one end of the analysis chip and extended along the axial direction of the parallel flow channel in the state where the analysis chip is inserted in the analysis device 1. In the void of the main body case 10, the light source unit 12b is disposed below the reaction flow channel located at the other end of the analysis chip and extended along the axial direction of the parallel flow channel in the state where the analysis chip is inserted in the analysis device 1. In the void of the main body case 10, each detection unit is disposed at the downstream side end in the insertion direction of the analysis chip so as to correspond to each reaction flow channel of the analysis chip in the state where the analysis chip is inserted in the analysis device 1.

There is no particular limitation on the size of the analysis device 1. The thickness of the analysis device 1 can be, for example, 10 mm ± 5 mm or about 8 mm. The length of the analysis device 1 in the longitudinal direction (the length of the analysis chip in the insertion direction) can be, for example, in the range from about 4 cm to 10 cm, and the length of the analysis device 1 in the width direction (the vertical direction to the insertion direction) can be, for example, in the range from about 1.5 cm to 8 cm.

FIG. 2 is a schematic view showing the direction of the light emitted from the light source units 12a and 12b in the analysis device 1. In the analysis device 1, the light source unit 12a and the light source unit 12b each are provided with the linear light source and the converter. In the analysis device 1, the linear light source of the light source unit 12a and the linear light source of the light source unit 12b are disposed such that the direction of the linear light emitted from the light source unit 12a is in the opposite direction to the direction of the linear light emitted from the light source unit 12b. Thus, in the analysis device 1, linear light is emitted from the linear light source of the light source unit 12a along the axial direction of the arrow A, the linear light in the direction of arrow A is converted into surface light by the converter of the light source unit 12a, and the surface light in the direction of arrow A' is emitted. On the other hand, in the analysis device 1, linear light is emitted from the linear light source of the light source unit 12b along the axial direction of the arrow B, the linear light in the direction of arrow B is converted into surface light by the converter of the light source unit 12b, and the surface light in the direction of arrow B' is emitted.

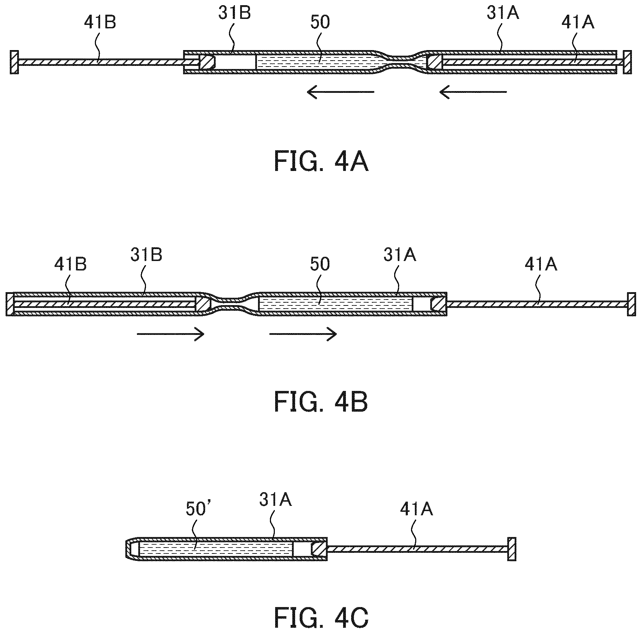

Next, an example of the second analysis chip of the present invention is described with reference to FIGs. 3 and 4.

FIG. 3 is a schematic view showing an example of an analysis chip of the present invention. FIG. 3 is a cross sectional view of an analysis chip 2. FIGs. 4A to 4C are schematic views each showing a part of the analysis chip 2. FIGs. 4A and 4B are cross sectional views each showing the method of mixing a sample and a reagent using the analysis chip 2. FIG. 4C is a cross sectional view showing the state where the analysis chip is separated.

As shown in FIG. 3, the analysis chip 2 includes a parallel flow channel 3 and a pair of parallel pistons 4A and 4B. The parallel flow channel 3 is composed of a plurality of flow channels 31 to 36 connected in parallel. The flow channels 31 to 36 each include the first and second syringe regions, and the tip of the first syringe region is connected to the tip of the second syringe region. The parallel flow channel 3 at the first syringe region side is referred to as a parallel region 3A at the first syringe region side, and the parallel flow channel 3 at the second syringe region side is referred to as a parallel region 3B at the second syringe region side. A reagent for target analysis is disposed in each of the flow channels 31 to 36 (not shown). The first parallel piston 4A to be inserted into the parallel region 3A at the first syringe region side includes: pistons 41A to 46A, in each of which a piston tip and a piston support are connected, respectively corresponding to the flow channels 31 to 36 in the parallel region 3A at the first syringe region side; and a piston connection site 47A that connects the pistons 41A to 46A. The second parallel piston 4B to be inserted into the parallel region 3B at the second syringe region side includes: pistons 41B to 46B, in each of which a piston tip and a piston support are connected, respectively corresponding to the flow channels 31 to 36 in the parallel region 3B at the second syringe region side; and a piston connection site 47B that connects the pistons 41B to 46B. FIG. 3 shows the state where a sample 50 is introduced in the parallel flow channel 3 for the sake of convenience. The present invention however is not limited thereto.

There is no particular limitation on the shape of each of the flow channels 31 to 36. The inner cross-sectional shape of the flow channel in the axial direction may be, for example, a circle including a perfect circle, an ellipse, or the like or a tetragon including a square, a rectangle, or the like. Preferably, the inner cross-section shape of the flow channel in the axial direction is a square. There is no particular limitation on the size of each of the flow channels 31 to 36. In the flow channels 31 to 36, the tip of the syringe region is thinner than other part of the syringe region (hereinafter, also referred to as "a cylindrical region"). The size of the cylindrical region in each of the flow channels 31 to 36 is, for example, about 2 mm × 2 mm.

The method of mixing a reagent and a sample using the analysis chip 2 is described with reference to FIGs. 3 and 4. First, the first parallel piston 4A or the second parallel piston 4B is taken out from the analysis chip 2, and a sample 50 is introduced into each of the flow channels of the parallel flow channel 3. Next, the parallel piston that has been taken out is again inserted into the position of the treatment of the parallel flow channel 3. Then as shown in FIGs. 4A and 4B, the first parallel piston 4A and the second parallel piston 4B are pushed alternately. Thereby, the sample 50 and the reagent in the parallel flow channel 3 are moved between the first syringe region and the second syringe region, whereby the sample 50 and the reagent are mixed. Next, the second parallel piston 4B is pushed to the end to introduce the mixture 50' of the sample and the reagent in the parallel flow channel 3 into the parallel flow channel 3A at the first syringe region side. Each piston of the first parallel piston 4A is inserted from one end of each flow channel located at the parallel region 3A at the first syringe region side which is to be used as the reaction detection unit. In this state, for example, the piston support of each piston of the first parallel piston 4A and the piston connection site 47A may be taken out while leaving the piton tip in the flow channel.

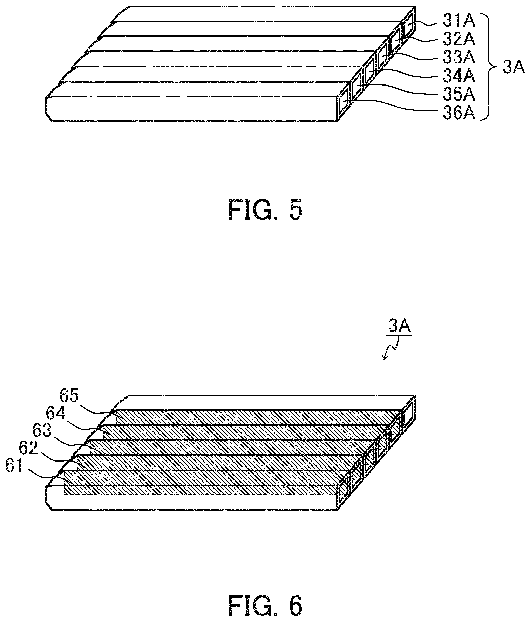

Furthermore, as shown in FIGs. 4 and 5, by cutting the section between the parallel region 3A at the first syringe region side, in which a reaction flow channel 31A is located, and the parallel flow channel 3B at the second syringe region, in which a flow channel 31B is located, the parallel flow channel at the first syringe region side 3A can be used as an analysis chip. In this case, the parallel region 3A at the first syringe region side separated from the analysis chip 2 can be subjected to the analysis device of the present invention as the reaction detection unit. In the present embodiment, the analysis chip 2 can be read as "a reaction system preparation chip", the parallel region 3A at the first syringe region side taken out from the reaction system preparation chip 2 can be read as "an analysis chip (or a reaction detection unit)", and each flow channel of the parallel flow channel 3A at the first syringe region side can be read as "a reaction flow channel" in the analysis chip.

An example of the analysis chip of the present invention obtained in this manner is shown in FIGs. 5 and 6.

FIG. 5 is a schematic view showing an example of an analysis chip of the present invention. As shown in FIG. 5, in the analysis chip 3A, plural reaction flow channels 31A to 36A are connected in parallel. One end of each of the plural reaction flow channels 31A to 36A is closed with the piston tip of each piston of the first parallel piston and the other end is also closed.

There are no particular limitations on the shape and the size of each of the reaction flow channels 31A to 36A. For example, the above description as to the shape and the size can be referred to. The length of each of the reaction flow channels 31A to 36A in the axial direction is not particularly limited, and, for example, can be determined according to a desired light path length. Specifically, the length is, for example, 2 cm to 5 cm or 2.5 cm to 5 cm.

As shown in FIG. 6, in the analysis chip 3A, side surfaces of the reaction flow channels 31A to 36A that are adjacent with each other each may have a light blocking section. This effectively prevents the detection unit that detects the reaction of a corresponding reaction flow channel from detecting the reaction of the reaction flow channel that is adjacent to the corresponding reaction flow channel in the analysis using the analysis device.

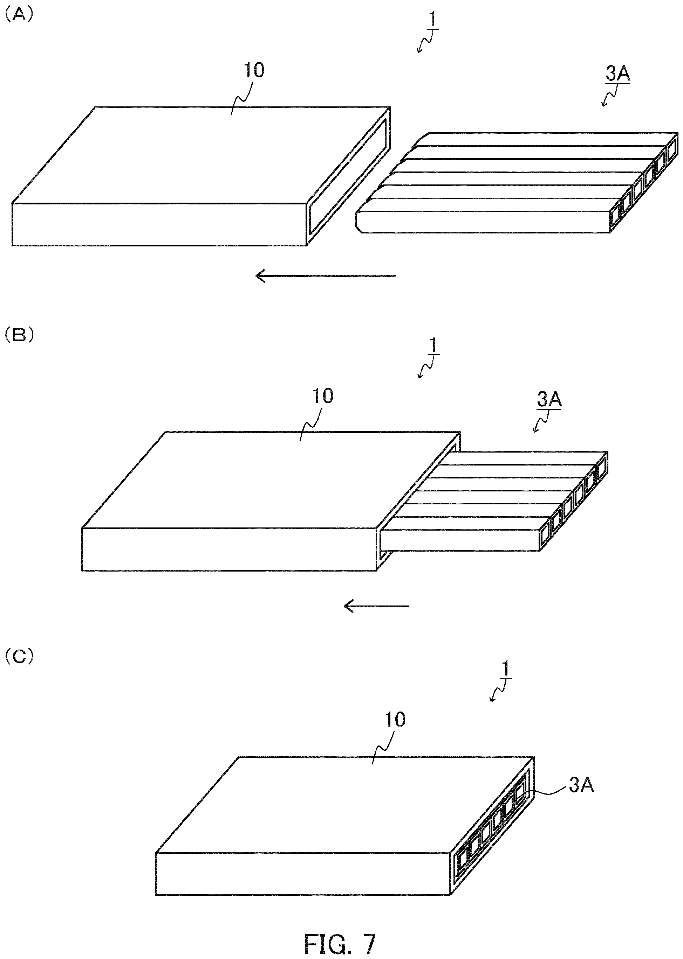

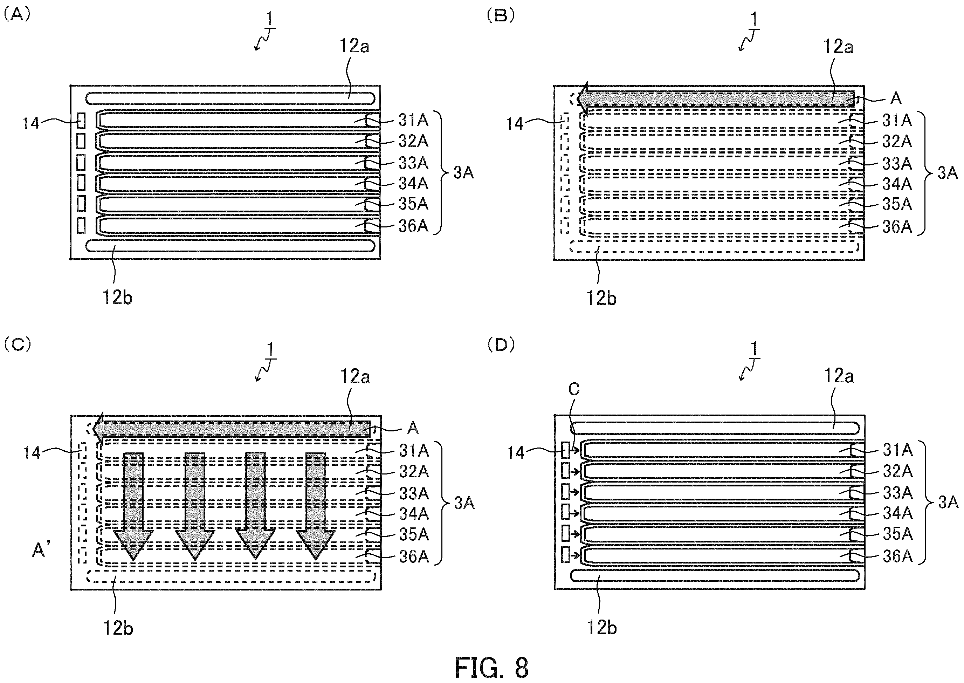

Next, the method of analyzing a sample using the analysis device and analysis chip of the present invention is described with reference to FIGs. 7 and 8.

(A) to (C) of FIG. 7 are schematic views showing the state of setting the analysis chip 3A into the analysis device 1. (A) to (D) of FIG. 8 are schematic views showing the state of irradiating the analysis chip 3A with light emitted from the light source units 12a and 12b of the analysis device 1 and detecting the reactions in the reaction flow channels by the detection units 14 of the analysis device 1 after the analysis chip 3A has set in the analysis device 1. While (A) to (D) of FIG. 8 are plan views showing the state where the analysis chip 3A is set in the analysis device 1, in order to make the state of light irradiation clearly understandable, the top surface of the analysis device is not shown and the top surface of the analysis chip 3A is exposed.

The analysis device 1 and the analysis chip 3A including a reaction system (reaction solution) obtained by mixing a sample and a reagent are prepared in the manner as described above. Then, as shown in (B) and (C) of FIG. 7, the analysis chip 3A is inserted into the analysis device 1 in the direction of the arrow shown in (A) of FIG. 7.

Then, the analysis chip 3A is heated by the heating unit 13 of the analysis device 1 to cause the sample and the reagent to react with each other in each of the reaction flow channels 31A to 36A. When the target is a nucleic acid, preferably, the reaction includes a nucleic acid amplification reaction using a primer, for example. The nucleic acid amplification reaction is not limited to a particular type, and examples of the nucleic acid amplification reaction include the PCR method, TMA method, NASBA method, LAMP method, ICAN method, RCA method, SDA method, HDA method, and SmartAmp method. Among them, an isothermal amplification method such as the SmartAmp method is preferable because it allows treatment at a constant temperature.

Then, the analysis chip 3A set in the analysis device 1 as shown in (A) of FIG. 8 is irradiated with linear light emitted from the light source unit 12a in the direction of arrow A as shown in (B) of FIG. 8. The linear light emitted from the light source unit 12a is converted into surface light, and the parallel surface at the upper side of the analysis chip 3A is irradiated with the surface light as shown in (C) of FIG. 8. Although it is not shown, linear light is also emitted from the light source unit 12b in the same manner, the light is converted into surface light, and the parallel surface at the lower side of the analysis chip 3A is irradiated with the converted surface light. Then, as shown in (D) of FIG. 8, each of the detection units 14 detects the reaction of a corresponding reaction flow channel (correspondence relationship is indicated by the arrow). The detection results obtained by the detection units 14 may be displayed on the analysis device 1 or on an external indicator (e.g., a personal computer, a cellular phone, a smartphone, a tablet, or the like) connected to the analysis device, for example.

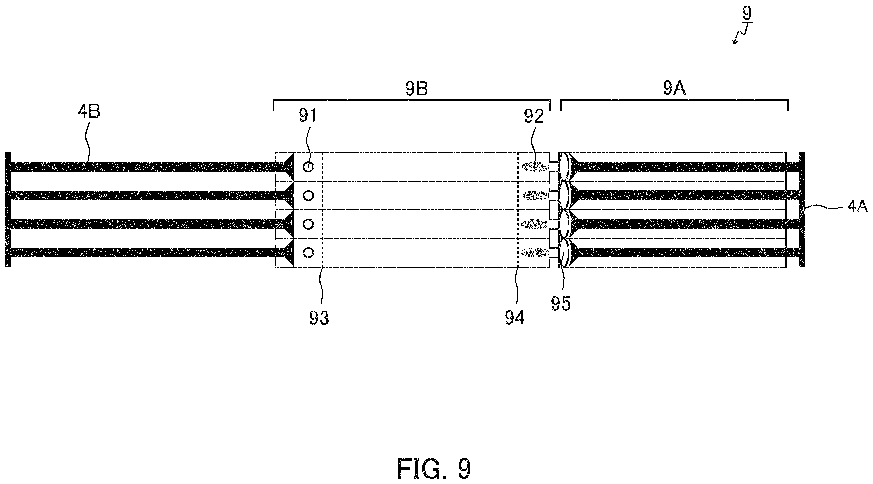

Next, another example of the analysis chip of the present invention and the analysis method using the same are described with reference to FIGs. 9 to 11.

FIG. 9 is a plan view schematically showing an example of the analysis chip of the present invention. As shown in FIG. 9, an analysis chip 9 includes a sample adding unit 9B and a reaction detection unit 9A each composed of a parallel flow channel, and a pair of parallel pistons 4B and 4A corresponding to the sample adding unit 9B and the reaction detection unit 9A. The sample adding unit 9B is in communication and is formed in one piece with the reaction detection unit 9A. Each flow channel of the sample adding unit 9B includes a sample feeding port 91 at one end, and a primer is disposed inside each flow channel at the other end as a reagent 92. A transparent lens-like movable piece 95 is disposed inside each flow channel of the reaction detection unit 9A. In FIG. 9, the line 93 indicates the upper limit of the amount of a sample to be added from the sample feeding port 91, and the line 94 indicates a stop line at the time of inserting the parallel piston 4B into the sample adding unit 9B.

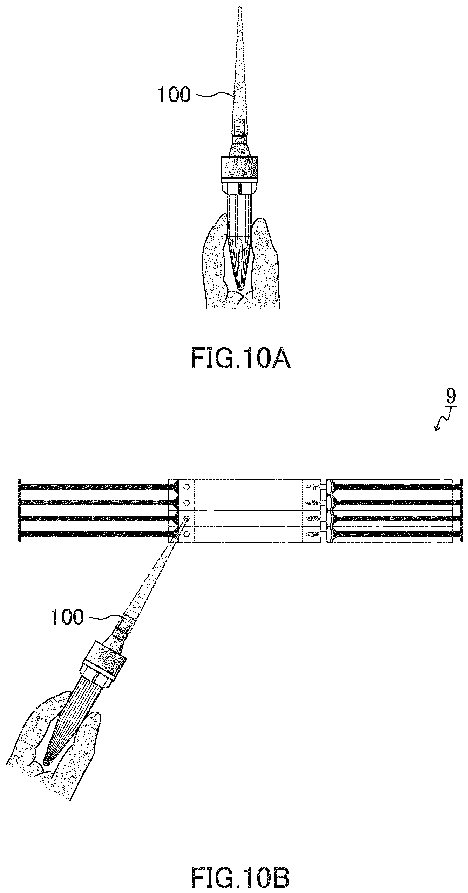

First, as shown in FIG.10A, a dropper 100 including a nozzle is prepared. In the nozzle of the dropper 100, a pretreated specimen and some of reagents for amplification (e.g. reaction buffer, enzyme, and the like) are mixed. Then, as shown in FIG. 10B, the mixed sample is injected to the sample feeding port 91 of the analysis chip 9 using the dropper 100. At this time, the mixed sample is injected to the line 93 of the sample adding unit 9B of the analysis chip 9.

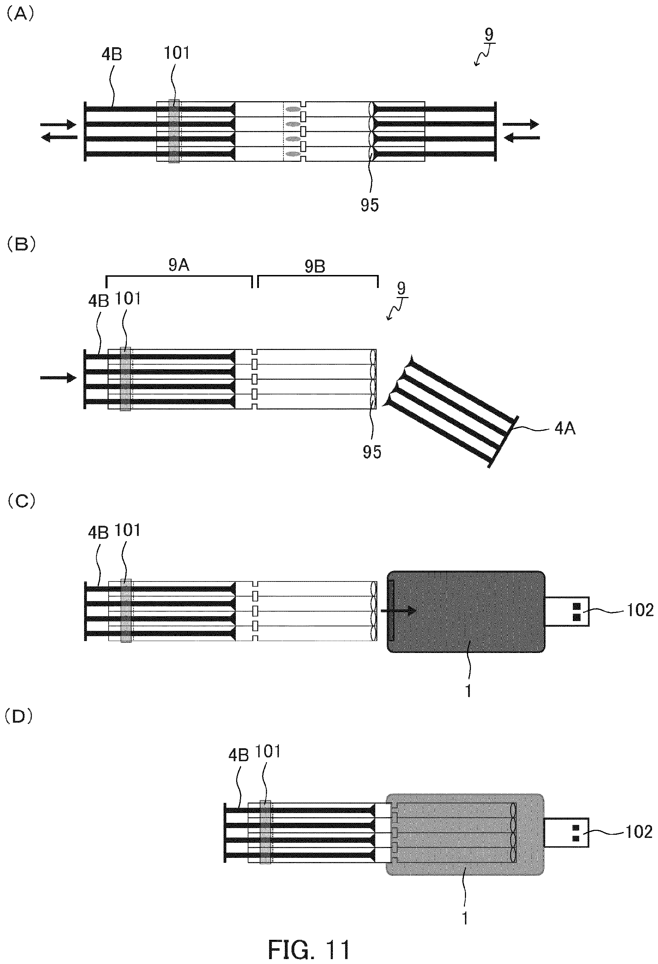

Next, as shown in (A) of FIG. 11, the sample feeding port 91 of the analysis chip 9 is sealed with a sticker 101, and then the parallel pistons 4A and 4B are pushed alternately to further mix the mixed sample and the primer in the analysis chip 9, thereby preparing a reaction solution. At this time, the movable piece 95 of the reaction detection unit 9A of the analysis chip 9 is moved in response to the movement of the parallel piston 4A. Then, as shown in (B) of FIG. 11, the parallel piston 4B is pushed to the line 94, and the parallel piston 4A is taken out. At this time, the movable piece 95 is stopped at the end of the reaction detection unit 9A.

Then, the reaction detection unit 9A of the analysis chip 9 is inserted into the analysis device 1 including a USB connector 102. Then, using the analysis device 1, the reaction detection unit 9A is heated to carry out a reaction, and the reaction is detected by a detection unit (not shown).

The invention of the present application was described above with reference to the embodiments and examples. However, the invention of the present application is not limited to the above-described embodiments and examples. Various changes that can be understood by those skilled in the art can be made in the configurations and details of the invention of the present application within the scope of the invention of the present application.

This application claims priority from Japanese Patent Application No. 2015-14849 filed on January 28, 2015. The entire subject matter of the Japanese Patent Application is incorporated herein by reference.

### Industrial Applicability

According to the present invention, for example, an analysis device can be downsized. Furthermore, the present invention allows, for example, an analyst to analyze a target in a sample easily by preliminarily mixing a sample and a reagent using the reaction system preparation chip of the present invention to prepare a reaction system, separating the analysis chip of the present invention from the reaction system preparation chip, and simply inserting the analysis chip into the analysis device of the present invention. Thus, for example, viral infection due to an influenza virus and the like and sexually transmitted diseases such as HIV, chlamydia, and the like can be analyzed easily. In particular, the present invention allows an analyst to analyze a target easily by himself/herself without going to an inspection agency such as a hospital or the like, for example, because the analysis device can be downsized.

### Explanation of Reference Numerals

1 analysis device
10 main body case
11 heat insulation section
12a, 12b light source unit
13 heating unit
14 detection unit
2, 9 analysis chip
9A reaction detection unit
9B sample detection unit
91 sample feeding port
92 reagent
93, 94 line
95 movable piece
3 parallel flow channel
3A parallel flow channel at first syringe region side (analysis chip or reaction detection unit)
3B parallel flow channel at second syringe region side
31 to 36 flow channel
31A to 36A reaction flow channel
31B flow channel
4A, 4B parallel piston
41A to 46A, 41B to 46B piston
47A, 47B connection site
50 sample
50' mixture
61 to 65 light blocking section
100 dropper
101 sticker
102 USB connector

## Claims

1. An analysis device into which an analysis chip is to be inserted for analyzing a reaction of a sample and a reagent in the analysis chip, the analysis chip to be inserted into the analysis device comprising a parallel flow channel in which plural reaction flow channels are connected in parallel, an axial direction of the parallel flow channel being a direction of inserting the analysis chip into the analysis device, the analysis device comprising:
a main body case being a housing, comprising:
an insertion opening into which the analysis chip is to be inserted; and
a void in communication with the insertion opening;
a heating unit that causes a sample to thermally react with a reagent in the analysis chip, the heating unit being disposed in the void at at least one of inner surfaces so as to face a parallel surface of the parallel flow channel of the analysis chip in a state where the analysis chip is inserted in the analysis device;
a light source unit that emits light to the analysis chip, the light source unit being disposed in the void at least above or below a reaction flow channel located at at least one end of the parallel flow channel of the analysis chip and extended along the axial direction of the parallel flow channel in the state where the analysis chip is inserted in the analysis device; and
a plurality of detection units that detect the thermal reactions of the samples and the reagents in the analysis chip, each detection unit being disposed in the void at a downstream side end in the insertion direction of the analysis chip so as to correspond to each reaction flow channel of the analysis chip in the state where the analysis chip is inserted in the analysis device.

2. The analysis device according to claim 1, further comprising:
a heat insulation section, wherein
the heating unit is disposed in the void of the main body case through the heat insulation section.

3. The analysis device according to claim 1 or 2, wherein
the light source unit comprises:
a linear light source; and
a converter that converts linear light into surface light,
wherein
the linear light source unit emits linear light along an axial direction of the reaction flow channel, and
the converter converts the linear light into surface light and emits the surface light.

4. The analysis device according to any one of claims 1 to 3, wherein the detection unit is CCD.

5. The analysis device according to any one of claims 1 to 4, wherein the analysis device is for nucleic acid analysis.

6. The analysis device according to any one of claims 1 to 5, further comprising:
a power supply; or
a connector to be connected to an external power supply.

7. The analysis device according to claim 6, wherein
the external power supply is a personal computer, a tablet, or a cellular phone.

8. An analysis chip to be inserted into an analysis device, comprising:
a parallel flow channel in which plural reaction flow channels are connected in parallel.

9. The analysis chip according to claim 8, wherein
the parallel flow channel has a translucent parallel surface, and
a side surface of each reaction flow channel that is in contact with an adjacent reaction flow channel in the parallel flow channel has light blocking ability.

10. The analysis chip according to claim 8 or 9, wherein
the parallel flow channel is configured to open and close openings at both ends of the reaction flow channels.

11. The analysis chip according to any one of claims 8 to 10, wherein
each reaction flow channel of the parallel flow channel comprises a condenser lens therein.

12. The analysis chip according to any one of claims 8 to 11, wherein
each reaction flow channel of the parallel flow channel comprises a reagent therein.

13. The analysis chip according to any one of claims 8 to 12, wherein
the reagent comprises an analysis reagent that reacts with a target in a sample.

14. The analysis chip according to claim 13, wherein
at least two reaction flow channels of the parallel flow channel comprise analysis reagents that react with different targets in the sample.

15. The analysis chip according to claim 13 or 14, wherein
the target is a nucleic acid, and
the analysis reagent comprises a reagent for nucleic acid amplification.

16. The analysis chip according to any one of claims 13 to 15, wherein the target is a nucleic acid, and
the reagent comprises a reagent for nucleic acid detection.

17. The analysis chip according to claim 16, wherein
the reagent for nucleic acid detection is a fluorescent reagent.

18. The analysis chip according to any one of claims 8 to 17, wherein the reagent comprises a pretreatment reagent of a sample.

19. An analysis chip to be inserted into an analysis device, comprising:
a parallel flow channel in which plural reaction flow channels are connected in parallel;
a pair of parallel pistons; and
a reagent, wherein
each flow channel of the parallel flow channel comprises a reagent therein,
each flow channel comprises:
a first syringe region; and
a second syringe region,
a tip of the first syringe region is connected to a tip of the second syringe region,
the flow channel can be cut in a vicinity of a connection site of the first syringe region and the second syringe region,
the pair of parallel pistons comprises:
a first parallel piston in which plural pistons are connected in parallel; and
a second parallel piston in which plural pistons are connected in parallel,
each piston of the first parallel piston can be inserted into each flow channel from an end side of the first syringe region of the parallel flow channel,
each piston of the second parallel piston can be inserted into each flow channel from an end side of the second syringe region of the parallel flow channel, and
after the sample and the reagent have mixed to prepare a reaction system, the parallel flow channel at a first syringe region side is usable as a reaction detection unit.

20. The analysis chip according to claim 19, wherein
in the parallel flow channel in which plural reaction flow channels are connected in parallel,
one of the parallel flow channel at the first syringe region side and the parallel flow channel at a second syringe region side is to be used as a flow channel for reagent arrangement and the other of the parallel flow channel at the first syringe region side and the parallel flow channel at the second syringe region side is usable as a flow channel for sample introduction.

21. The analysis chip according to claim 19 or 20, wherein
the parallel flow channel at the first syringe region side to be used as the reaction detection unit has a translucent parallel surface, and
a side surface of each flow channel that is in contact with an adjacent flow channel in the parallel flow channel has light blocking ability.

22. The analysis chip according to any one of claims 19 to 21, wherein after the sample and the reagent have mixed to prepare a reaction system, by cutting the parallel flow channel in a parallel direction, the parallel flow channel at the first syringe region side is usable as a reaction detection unit.

23. The analysis chip according to any one of claims 19 to 22, wherein
the parallel flow channel at the first syringe region side to be used as the reaction detection unit comprises a condenser lens therein.

24. The analysis chip according to any one of claims 19 to 23, wherein the reagent comprises a pretreatment reagent of a sample.

25. The analysis chip according to any one of claims 19 to 24, wherein
the reagent comprises an analysis reagent that reacts with a target in a sample.

26. The analysis chip according to claim 25, wherein
at least two flow channels of the parallel flow channel comprise analysis reagents that react with different targets in the sample.

27. The analysis chip according to claim 25 or 26, wherein
the target is a nucleic acid, and
the analysis reagent comprises a reagent for nucleic acid amplification.

28. The analysis chip according to any one of claims 25 to 27, wherein
the target is a nucleic acid, and
the analysis reagent comprises a reagent for nucleic acid detection.

29. The analysis chip according to claim 28, wherein
the reagent for nucleic acid detection is a fluorescent reagent.

30. The analysis chip according to any one of claims 19 to 29, wherein
each piston of the first parallel piston to be inserted into the parallel flow channel at the first syringe region side comprises an optical fiber.

31. An analysis kit comprising:
the analysis device according to any one of claims 1 to 7; and
the analysis chip according to any one of claims 8 to 30.

32. An analysis method of a sample comprising the following steps:
introducing a sample into the analysis chip according to any one of claims 8 to 30;
mixing the sample and the reagent in the analysis chip;
inserting the analysis chip into the analysis device according to any one of claims 1 to 7;
causing the sample to thermally react with the reagent in the analysis chip by the heating unit of the analysis device; and
detecting the reaction in the analysis chip by the detection unit of the analysis device.

33. The analysis method according to claim 32, wherein
in the introduction step,
the first parallel piston is inserted from an end of the parallel flow channel at the first syringe region side,
a sample is introduced from an end of the parallel flow channel at the second syringe region side, and
after the sample has introduced, the second parallel piston is inserted from the end of the parallel flow channel at the second syringe region side,
or
the second parallel piston is inserted from an end of the parallel flow channel at the second syringe region side,
a sample is introduced from an end of the parallel flow channel at the first syringe region side, and
after the sample has introduced, the first parallel piston is inserted from the end of the parallel flow channel at the first syringe region side.

34. The analysis method according to claim 32 or 33, wherein
by alternately pushing the first parallel piston and the second parallel piston, the sample and the reagent are mixed in each flow channel of the parallel flow channel.

35. The analysis method according to any one of claims 32 to 34, wherein
after the mixing step and before the insertion step, by cutting the parallel flow channel of the analysis chip according to any one of claims 19 to 30 in a parallel direction, the parallel flow channel at the first syringe region side is separated as a reaction detection unit, and the reaction detection unit is inserted into the analysis device as the analysis chip.

36. The analysis method according to any one of claims 32 to 35, wherein an analysis target is a nucleic acid.

37. The analysis method according to any one of claims 32 to 36, wherein a sample is a biological sample.

38. The analysis method according to claim 37, wherein
the biological sample is at least one selected from the group consisting of blood, blood plasma, and blood serum.

39. The analysis method according to any one of claims 32 to 38, wherein an analysis target is a virus or a bacterium.
